# EUROPEAN PATENT SPECIFICATION

(11) **EP 3 930 644 B1**
(45) Date of publication and mention of the grant of the patent: **25.03.2026**
(21) Application number: 20708580.4
(22) Date of filing: 21.02.2020
(51) Int. Cl.: A61F 5/445

(54) **A VALVE FOR A UROSTOMY APPLIANCE**
VENTIL FÜR EIN UROSTOMIEGERÄT
VANNE POUR APPAREIL D'UROSTOMIE

(30) Priority: 28.02.2019 GB 201902745; 28.02.2019 GB 201902746; 24.01.2020 GB 202001052
(43) Date of publication of application: 05.01.2022
(73) Proprietor: Salts Healthcare Limited, West Midlands B7 4AA (GB)
(72) Inventor: ALLEN, Marcus, Aston, Birmingham West Midlands B7 4AA (GB)
(74) Representative: Forresters IP LLP
(86) International application number: PCT/GB2020/050417
(87) International publication number: WO 2020/174220

(56) References cited:
- WO-A1-2009/012304
- WO-A1-2018/098242
- US-A- 4 055 179
- US-A- 4 306 705
- US-A1- 2015 190 272

## Description

Embodiments of the present invention relates to a valve for a urostomy appliance.

Urostomy appliances are well known in the field. They are typically attached to a patient via an adhesive wafer that extends around the patient's stoma with adhesive and provide a collecting chamber to collect waste (mostly liquid waste) exiting the stoma. A mechanism for draining the collecting chamber is often provided - typically, these are in the form of a tap or bung, which allow the patient to open an outlet from the urostomy appliance and drain the contents, for example, into a toilet.

WO2018/098242 discloses a drainable ostomy system including an ostomy pouch having an internal collection area and an outlet for draining contents form the collection area, and an interface assembly. US4055179 discloses a two-piece valve including a first tubular member having a flow passageway therethrough with a tapered valve seat formed at one end of the flow passageway. US 4.055.179 discloses a two-piece valve including a first tubular member having a flow passageway therethrough with a tapered valve seat formed at one end of the flow passageway

According to an aspect of the present invention we provide a valve for a urostomy appliance including: a body for connection to the urostomy appliance formed from first and second separate body parts, which are connected or connectable to each other, an inlet and an outlet, connected by a flow path, the inlet being provided by the first body part, and characterised by: a blocking member which is received in an opening provided by the second body part, said blocking member being moveable between a closed position, in which the blocking member blocks the inlet, such that liquid cannot flow through the flow path to the outlet, and an open position in which the inlet is open, such that liquid is permitted to flow through the flow path to the outlet, and a seal device extends around the blocking member and between the blocking member and the body, which seal device prevents or at least inhibits liquid exiting the valve through the opening in the body and between the body and the blocking member.

Further features of the present invention are outlined by the appended claims.
Figures 1 and 2 show a urostomy appliance in accordance with an embodiment of the present invention,
Figure 3 shows a valve in accordance with an embodiment of the present invention,
Figures 4 to 6 show various views of a valve in an open position in accordance with an embodiment of the invention,
Figure 7 shows a valve in accordance with an embodiment of the present invention,
Figure 8 shows a valve in accordance with an embodiment of the present invention,
Figure 9 shows a valve in accordance with an embodiment of the present invention,
Figure 10 shows an exploded view of a valve in accordance with an embodiment of the present invention,

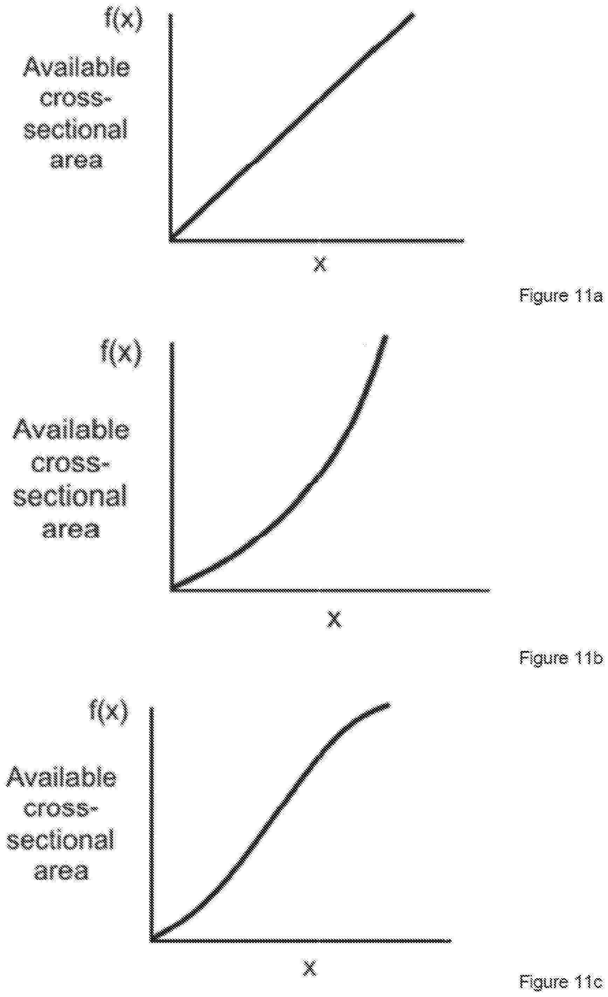

Figures 11a, b, c illustrate the available cross-sectional area of different inlet shapes,
Figure 12 illustrates a connector for connecting to a valve,
Figure 13 illustrates a valve in accordance with an embodiment of the invention,
Figure 14 illustrates a cross-sectional view of a valve,
Figure 15a illustrates a cross-sectional view of a part of the valve, and
Figure 15b illustrates a cross-sectional view of a part of the valve.

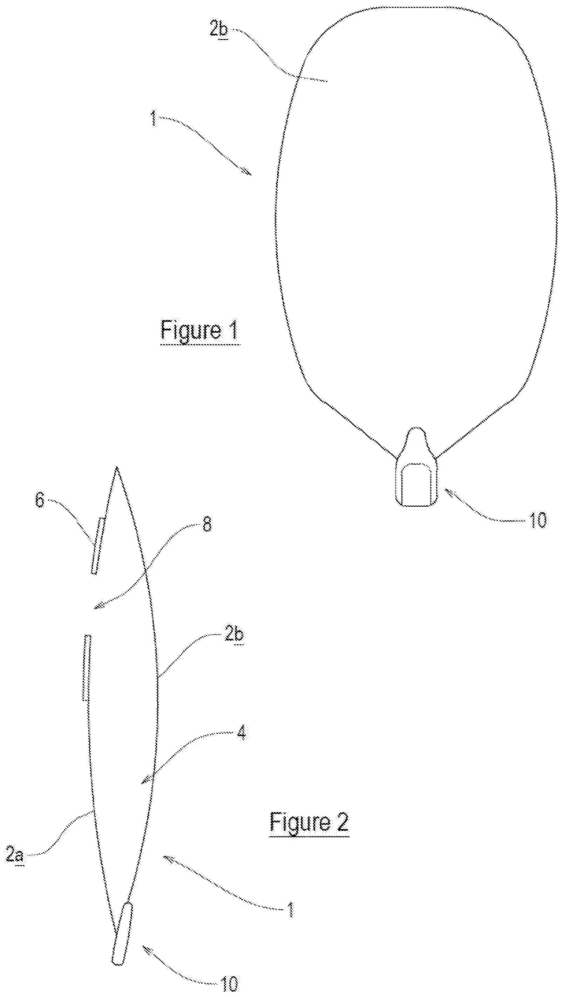

With reference to figures 1 and 2, particularly, a urostomy appliance 1 is illustrated. The urostomy appliance 1 includes first and second walls 2a, 2b which are connected together (for example, via a heat weld) to form a waste collecting cavity 4. The first wall 2a is attached to an adhesive wafer 6. An aperture (known as a stoma receiving opening 8) extends through both the first wall 2a and the adhesive wafer 6 to provide an entrance to the waste collecting cavity 4.

A valve 10 (described in more detail below) is attached to the second wall 2b of the urostomy appliance 1. The valve 10 communicates with the waste collecting cavity 4 and has an open and a closed position, in which waste is or is not permitted to flow through the valve 10 (i.e. flow out of the waste collecting cavity 4).

In use, the patient attaches the adhesive wafer 6 around their stoma. Waste liquid (for example, urine and/or blood and/or other body fluids) exits the body, via the stoma, and flows through the stoma receiving opening 8 and is collected in the waste collecting cavity 4. The valve 10 is selectively used to permit the waste which is collected in the waste collecting cavity 4 to flow out of the appliance 1, through the valve 10 (so that, for example, the appliance 1 can be emptied of some/most/all of the contents).

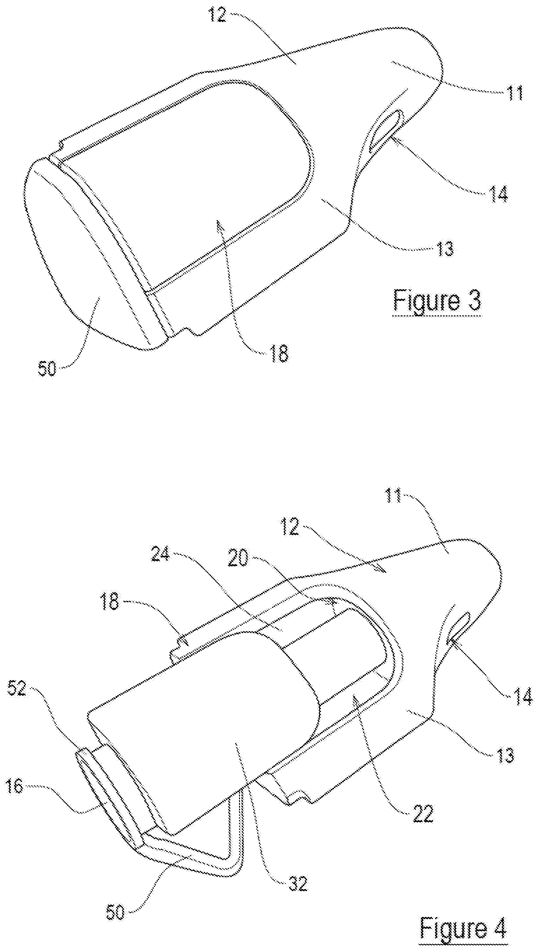

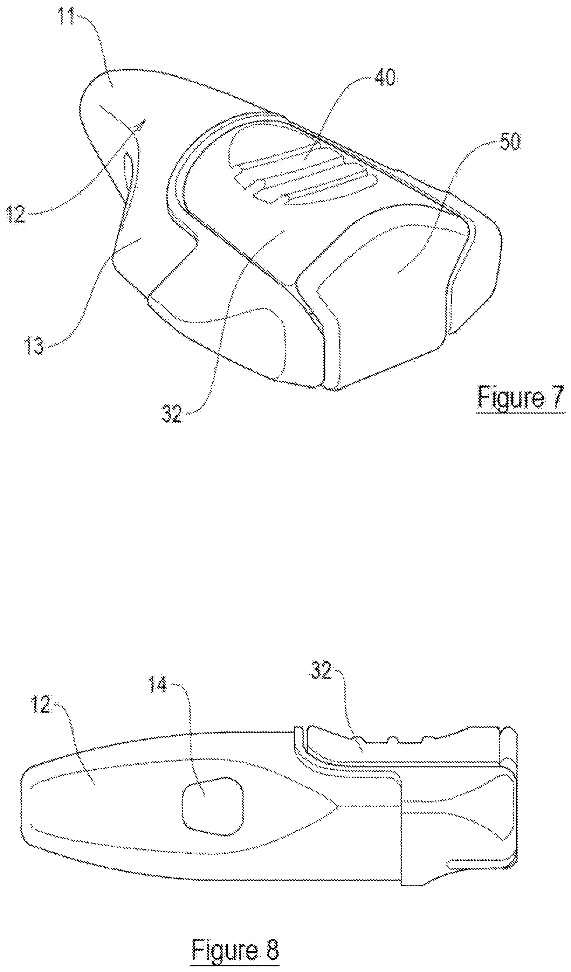

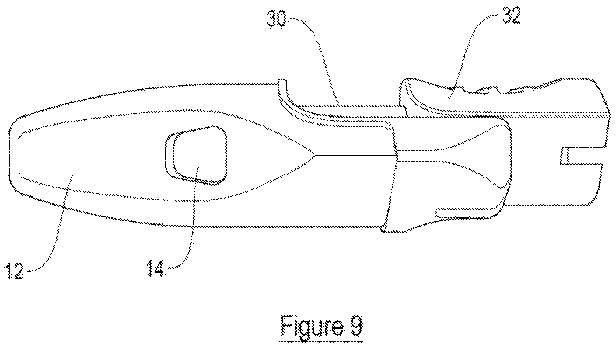

Features of the valve 10 are shown in more detail in figures 3 to 10. The valve 10 includes a body 12 for connection to the urostomy appliance 1, an inlet 14, and an outlet 16, which are connected by a flow path, and a blocking member 18, which is supported by the body 12.

In some embodiments (see particularly figures 3, 4, 7 and 10), the body 12 includes a tip that has a rounded and narrowed end 11 and a wider base part 13. In this case, the inlet 14 is defined by the body 12 (for example, the inlet 14 is formed adjacent the narrowed end of the body 12 and communicates with a body passage 20 that extends through part of the body 12. In some embodiments, the body 12 includes a further inlet (i.e. there are two inlets 14 in total). The further inlet is located on the opposing "side" of the body 12. In other words, the further inlet is positioned so that it also communicates with the body passage 20. In such a design, the further inlet is opened and closed synchronously with the inlet 14 (by the movement of the blocking member 18). It should be appreciated that two such inlets 12 are not necessarily required and there may be fewer or more as desired in a specific design.

In some embodiments, the inlet (or inlets) 14 are shaped in one of a tear-drop, egg/oval, trapezoidal, pentagonal or kite shape when viewed in a direction liquid passes therethrough.

In the illustrated embodiment, the body 12 also includes a channel 22. The channel 22 extends away from an entrance/exit of the body passage 20 and provides support for the blocking member 18 (the function of the channel 22 is described in more detail below).

The body 12 is connected to the urostomy appliance 1 by a heat weld (not shown) around the body 12, such that the inlet 14 communicates with the waste collecting cavity 4 (for example, the heat weld may extend across the base part 13 of the body 12, such that the narrowed end 11 and inlet 14 is in the urostomy appliance 1 / waste collecting cavity 4). It should be appreciated that the body 12 need not be heat welded necessarily. The body 12 may be attached to the appliance 1 in another manner that permits the inlet 14 to communicate with the waste collecting cavity 4.

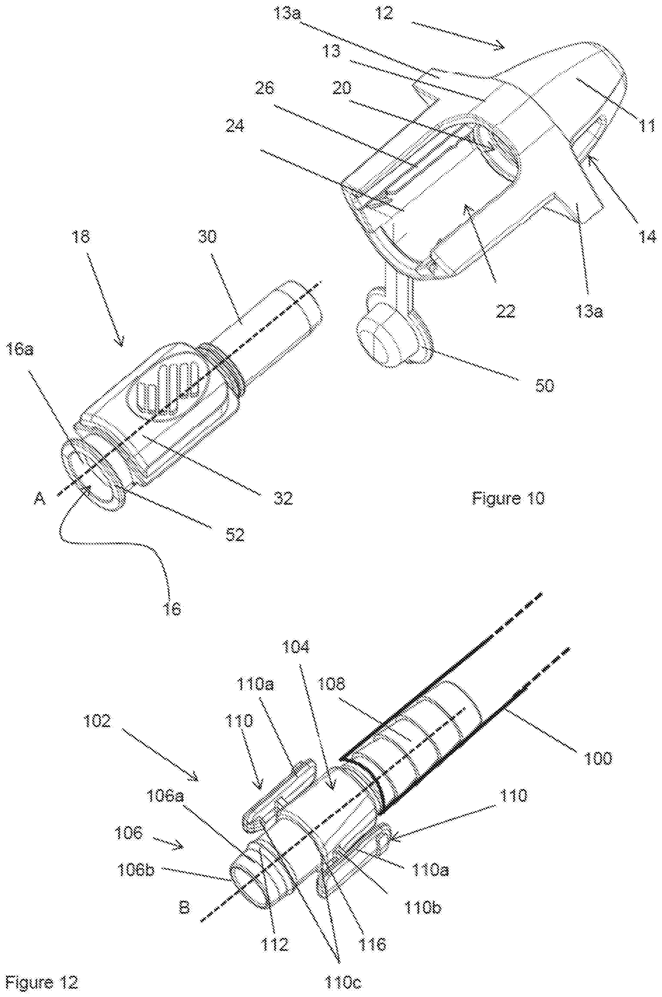

The embodiment illustrated in figure 10 shares the features of the valve 10 described in relation to the other figures (but only some features are labelled explicitly). In this case, the base part 13 of the body 12 includes two projections 13a which extend outwards from the sides of the base part 13. Each projection 13a includes an upper surface and a lower surface, which are inclined with respect to each other so that the edge furthest from the base part 13 is narrow (in the illustrated version, the upper and lower surfaces form a point at the edge furthest from the base part 13). The side projections 13a improve the manufacturing process by making the heat weld between the body 12 and urostomy appliance 1 easier to form because the films (or the urostomy appliance 1) that form the seal around the valve 10 (that seal around the entire base part 13) form a smoother, less extreme curve, around the projections 13a than if there were no projections present. It should be appreciated that any embodiment may include this feature, as desired.

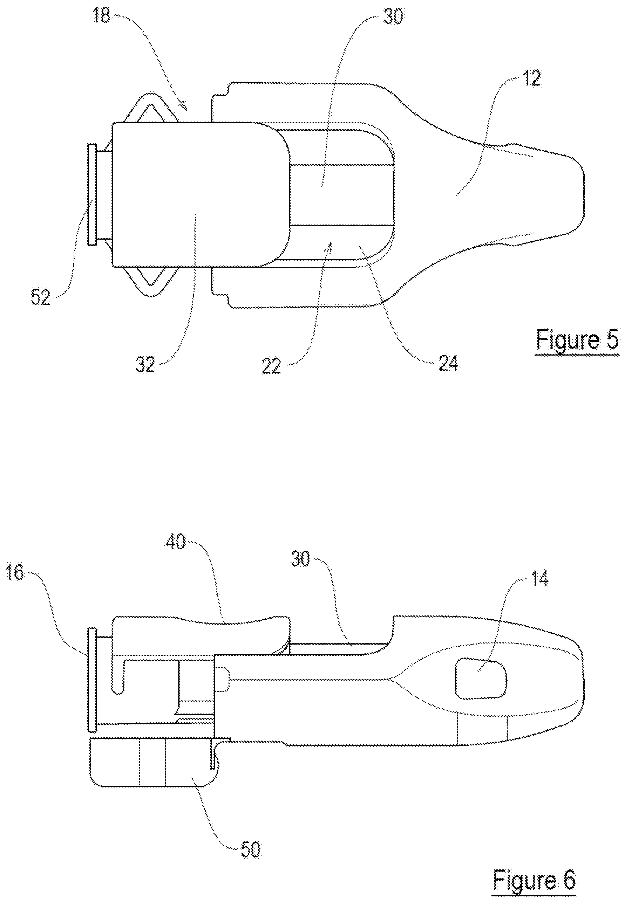

In some embodiments, a main part of the body 12 (i.e. the top and wider base part forming the body passage 20 and the channel 22) is made of a first material. The body 12 also includes a guide channel 24 (see figures 4 and 5), which is integrally formed (in this case, along a base of the channel 22). The guide channel 24 is made of a second material. The second material is more rigid than the first material.

The first material may be relatively pliable which means the body 12 is deformable and can be distorted reasonably easily. This allows the body 12 to be attached and sealed to the urostomy appliance 1 relatively easily but may make the body 12 more prone to damage if it is handled carelessly by a user (for example, if the valve 10 is squashed/distorted repeatedly). By including a second material to form a guide channel 24 that is more rigid than the rest of the body 12 may reduce distortion of the body 12 and may result in less damage being inflicted on the valve 10 during its useable life. It should be appreciated that this configuration may only be necessary if such the first material used for the body 12 is pliable / distortable. In some embodiments, the body 12 may be made from a first material that provides enough rigidity for the valve to operate as desired. It should also be appreciated that the first and second materials may be the same material but formed differently to provide different physical characteristics (e.g. two forms of PTFE).

It should be appreciated that the body 12 and the blocking member 18 may be manufactured using an overmoulding process. The blocking member 18 and/or the body 12 may be overmoulded with a rubber or rubber-like material.

The blocking member 18 is supported by the body 12 and is moveable between a closed position and an open position (as illustrated in figures 3 and 4, respectively). In the illustrated example, the blocking member 18 is generally linearly moveable between its closed open positions (and vice versa), but this does not necessarily have to be the case.

In the illustrated embodiments, the blocking member 18 includes a blocking portion 30 and a user operable part 32. A passage extends through the blocking member 18 (in this case, through both the blocking portion 30 and the user operable part 32) to connect to the outlet 16. In the present example, the opening extends substantially centrally, but this need not be the case.

When the valve 10 is assembled, and the blocking member 18 is in its closed position, the blocking portion 30 is received in the body passage 20 and the user operable portion 32 is received by the channel 22 (see figure 3). Thus, the passage through the blocking member 18 communicates with the body passage 20 through the body 12 to provide the flow path (between the inlet 14 and the outlet 16) through the valve 10 (whether the flow path is open for liquid to flow through depends on the position of the blocking member 18 in the body passage 20).

In some embodiments, the blocking member 18 is received and supported by the guide channel 24. The channel 24 prevents or at least inhibits movement of the blocking member 18 that is not generally linear with respect to the body 12. In some embodiments, the guide channel 24 inhibits non-coaxial movement of the blocking member 18 when the blocking member 18 is moved between its closed and open positions.

In some embodiments, the guide channel 24 includes a formation 26 (see figure 10) that engages a corresponding formation on the blocking member 18. Such a formation 26 and corresponding formation may provide additional guidance for the blocking member 18 as it is moved between its open and closed positions. It should be appreciated that such a formation and corresponding formation may not be present.

In some embodiments, the formation 26 of the guide channel includes one or more formations, each of which extends along the guide channel 24 in the direction of movement of the blocking member 18. The corresponding formation on the blocking member 18 includes at least one further formation. When the respective formations engage with each other (i.e. as the blocking member 18 moves), they inhibit any movement apart from generally linear movement of the blocking member 18. The formations could be axially extending projections/ridges and may be positioned on the base of the guide channel 24 or on each side of the guide channel 24, for example.

In some examples, the user operable portion 32 includes a depression 40 to aid user control. In some examples, the depression 40 also has a textured surface. These features aid user control by providing an area for a finger or thumb to be placed and provide the force required to slide open the valve 10.

In some embodiments, the valve 10 has an indicator that allows a user to feel when the valve 10 is in a fully open position. For example, the channel 22 may include an upward projection. When the blocking member 18 is moved to its open position (i.e. linearly outwards from the body 12) a part may pass over the projection and provide an indication that the blocking member 18 is in its fully open position. This allows the user to be confident that the valve 10 is fully open.

Likewise, another indicator could be used to inform the user when the valve 10 is in a closed position. This would allow a user to be confident that the valve 10 is closed and will not leak, for example.

In some embodiments, the valve 10 includes a cover member 50. The cover member 50 is moveable between an open position (see figure 6) and a closed position (see figure 3). When the cover member 50 is in its open positon, liquid is permitted to flow through the outlet 16, and when the cover member 50 is in its closed position, liquid is prevented or at least inhibited from flowing through the outlet 16.

In some embodiments, the cover member 50 pivots downwardly and towards a user wearing the urostomy appliance 1 when it is moved to its open positon when the cover member 50 is moved or moves to its open position. In other words, when the cover member 50 is in its open position it may be located between the user and the blocking member 18.

In some embodiments, the cover member 50 includes a holding formation (not shown) and the blocking member 18 includes a corresponding formation 52. When the holding formation engages the corresponding formation 52 the cover member 50 is held in its closed position (over the end of the blocking member 18 and sealing the outlet 16 in order to prevent or at least inhibit drips of liquid from exiting the outlet 16).

In some embodiments, the cover member 50 may be biased to its open position. In other words, once the holding formation and the corresponding formation 52 are disengaged from each other the cover member 50 may automatically move away from the outlet 16.

In some embodiments, the cover member 50 is moved due to movement of another part of the valve 10. In an example, as the blocking member 18 is moved towards its open position, the blocking member 18 effects movement of the cover member 50 to its open position. In such embodiments, the cover member 50 may have no biasing or may be biased towards a closed position (such that opening of the blocking member 18 controls the opening of the cover member 50).

The cover member 50 is attached to the body 12. In some embodiments, the cover member 50 includes a resiliently biased and flexible connection portion that extends to form the connection / attachment to the body 12.

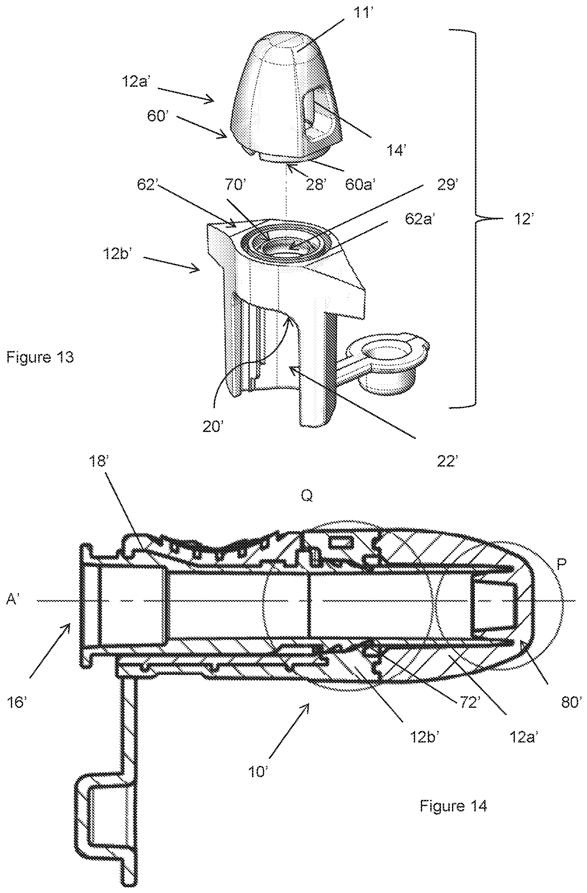

Figures 13 to 15b illustrate a further configuration of valve, which is described in detail below. Where features are the same, or similar, as those already described, the reference numerals are altered to include a prime symbol ('). For example, valve 10 becomes valve 10'. It should be appreciated that any features not specifically described in relation to this configuration of the valve 10' but described in the description elsewhere in relation to the other figures / configurations should be considered features that may be present in this configuration of valve 10' unless it is explicitly mentioned that such a combination of features is not possible.

In some embodiments, the valve 10' has a body 12', which is formed from a first body part 12a' and a second body part 12b'. The first and second body parts 12a', 12b' are separate or discrete parts, which are connected or connectable to each other to make up the body 12'.

In this embodiment, the inlet 14' is provided on the first body part 12a'. As described above, this inlet 14' communicates with the waste collecting cavity 4 of the urostomy appliance 1. The first body part 12a' has a first end 11' (which in this case is rounded and narrowed) and a second end (which in this case is wider than the first end 11'). The second end has a second opening 28'. A first portion of the body passage 20' extends from the second opening 28' in the second end of the first body part 12a' to the inlet 12' (and towards the first end 11' of the first body part 12a')).

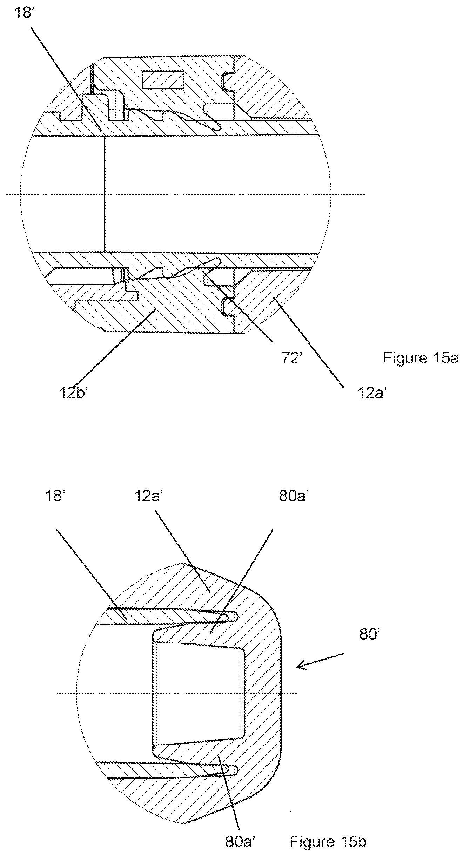

In some embodiments (see particularly figure 14 and figure 15b), the first body part 12a' includes a receiving part 80', which receives an end of the blocking member 18' when the blocking member 18' is in the closed position. The receiving part 80' is located in the first portion of the body passage 20' (i.e. in the internal passage of the first body part 12a'). The receiving part 80' includes a pair of projections 80a' that extend outwardly from an end of the first body part 12a'. The purpose of the receiving part 80' is to provide a (snug) friction fit with the end of the blocking member 18', when the blocking member 18' is in its closed position. The functionality provided is to improve the closing of the flow path between the inlet 14' and the outlet 16' when the blocking member 18' is in its closed position. In other words, any liquid that flow into the inlet 14' cannot exit through the passage in the blocking member 18'. In some embodiments, the receiving part 80' may be made from a resilient material to provide an improved seal between the first body part 12a' and the blocking member 18' (and, more specifically, improved sealing between the body 12' and the an end of the blocking member 18' that provides an opening that connects to the outlet 16' (and provides part of the flow path)).

As described in relation to other embodiments, the body 12' has an opening and a body passage 20'. In this example, the body passage 20' is split between the first and second body parts 12a', 12b', with the first portion of the body passage 20' being through the first body part 12a' and a second portion of the body passage 20' being through the second body part 12b'). The second body part 12b' provides the entrance or opening to the body passage 20' (which, when the body 12' is formed, received the blocking member 18'). The second portion of the body passage 20' extends from the opening to a second opening 29' in the second body part 12b'

As the first and second body parts 12a', 12b' are connected together to form the body 12', the body passage 20' extends away from the entrance / opening in the second body part 12b', through the second body part 12b' and into the first body part 12a', so that the body passage 20' can communicate with the inlet 14'. In other words, the first body part 12a' and the second body part 12b' are connected at their respective second openings 28', 29', so that when the body 12' is formed, the first and second portions of the body passage 20' are connected together.

In some embodiments, the second body part 12b' also includes the channel 22', which extends away from the entrance to the body passage 20' (and away from the second opening 29' of the second body part 12b').

Examples of how the first and second body parts 12a', 12b' are connected together will now be described. In some embodiments, the first body part 12a' includes a first connection formation 60' and the second body part 12b' includes a corresponding second connection formation 62'. When the first and second body parts 12a', 12b' are connected together to form the body 12', the first and second connection formations 60', 62' engage with each other.

In some embodiments, the first connection formation 60' includes a projection 60a' and the second connection formation 62' includes a recess 62a'. Thus, when the first connection formation 60' engages the second connection formation 62', the projection 60a' is received in the recess 62a'. It should be appreciated that the second connection formation 62a' could include a projection and the first connection formation 60' the recess without altering the functionality of the connection between the first and second body parts 12a', 12b'.

In some embodiments, the first and second connection formations 60', 62' engage each other with a snap or friction fit engagement. Alternatively or additionally, adhesive may be applied to one or both of the first and second connection formations 60', 62' before they are brought into engagement. Using adhesive may be advantageous because it provides improved sealing and / or a more permanent connection between the body parts 12a', 12b' and, therefore, the valve may operate better (i.e. a reduction in the chance of leakage, etc.).

The blocking member 18' is received in the opening in the second body part 12b' and extends along the body passage 20'. In other words, the blocking member 18' extends through the second body part 12b' and into the first body part 12a'. Thus, in the same manner as those valve configurations described above, the blocking member 18' is being moveable between a closed position and an open position. In the closed position, the blocking member blocks the inlet 14', such that liquid cannot flow through the flow path to the outlet 16'. In the open position (in which the inlet 14' is open), the blocking member 18' is moved (to unblock the inlet 14') such that liquid is permitted to flow through the flow path to the outlet 16'.

In some embodiments, the valve 10' further includes a seal device 70' which prevents or at least inhibits liquid exiting the valve 10' through the opening in the body 12' and between the body 12' and the blocking member 18'. In some embodiments, the seal device 70' extends around the blocking member 18' and between the blocking member 18' and the body 12'. In other words, the seal device 70' is connected to or provided on an internal surface of the body 12' (and extends around the periphery of the blocking member 18' that extends through / into the body passage 20').

In some embodiments, the seal device 70' is located on the second body part 12b'. In the illustrated case, the seal device 70' is positioned at an entrance / opening of the second body part 12b' (and may be adjacent where the second body part 12b' connects to the first body part 12a'). Thus, the seal device 70' may be positioned adjacent the second connection formation 62'.

It should be appreciated that the seal device 70' could be provided on the first body part 12a'. In which case, the seal device 70' could be located at the entrance of the first body part 12a' (where it connects to the second body part 12b') and / or adjacent to the first connection formation 60'.

The seal device 70' is visible particularly in figure 14 and 15a (which is an enlarged view of the part encapsulated by "Q" in figure 14). In some embodiments, the seal device 70' includes a projection 72' that extends radially inwards. An axis A' extends substantially centrally through the body 12' and the blocking member 18' (i.e. generally centrally of and parallel with a substantial part of the flow path through the valve 10'). In other words, the projection 72' extends inwards from the internal surface of the body 12' towards the blocking member 18' (and, in use, will contact an exterior / outer surface of the blocking member 18', so that a good seal can be provided).

In some embodiments, the seal device 70' also includes a portion that extends axially along the axis A'. The portion may extend away from the opening of the second body part 12b'. This may also be towards a distal end of the first body part 12a' (the narrowed end 11') when the first and second body parts 12a', 12b' are connected together.

In some embodiments, the seal device 70' includes the projection 72' which extends both inwardly, towards the blocking member 18', and away from the opening in the second body part 12b'.

In some embodiments, the seal device 70' (in this illustrated case the projection 72') is formed from a resilient material. This allows the material to flex against the exterior / outer surface of the blocking member 18' and provide a seal that prevents liquid from flowing out of the valve 10' where the blocking member 18' is received (i.e. through the opening). As the blocking member 18' is moved between the open and closed positions, the seal device 70' flexes against the surface and maintains the seal.

Use of the valve 10 will now be discussed with reference to the features that have already been outlined above. In the closed position, the blocking member 18 blocks the inlet 14, such that fluid cannot flow through the valve 10. More specifically, when the valve 10 is in a closed position, the blocking portion 30 is positioned in the body passage 20 and closes off (i.e. seals) the inlet 14 (and liquid cannot flow through the inlet 14 and into the valve 10).

In the open position, the inlet 14 is open, such that fluid is permitted to pass through the valve 10. More specifically, when the valve 10 is in the open position, the blocking portion 30 is moved to an "outer" position, the inlet 14 communicates with the opening through the blocking member 18. In this configuration, liquid is permitted to exit the valve 10 via the outlet 16. It should be appreciated that such an "open" position refers to a fully open position in which the inlet 14 is open to its fullest extent (so the blocking portion 30 blocks the inlet 14 to the least extent possible) to allow a maximum flow rate of liquid to flow through the valve.

There is a range of partially open positions in which the blocking member 18 can be positioned which will result in a different flow rate of liquid being permitted to flow through the inlet 14 (due to it being partially open) and, thus, through the valve 10. In other words, the inlet 14 has a (total) cross-sectional area that is dictated by its size and shape. The inlet 14 also has an "available cross-sectional area" which is the available area (during use) through which liquid may flow. While the total cross-sectional area of the inlet 14 will not change substantially (it may change slightly through use of the valve 10 and general wear), the size of the available cross-sectional area depends on the position of the blocking member 18 (until the blocking member 18 reaches the fully open position and the entirety of the inlet 14 is open and available for liquid to flow through).

The inlet 14 is shaped such that as the blocking member is moved between a position in which a flow path is initially opened and a fully open position, the rate of change of the permitted flow rate of liquid permitted through the valve 10 increases with non-linear proportionality as the blocking member 18 is moved (although it should be appreciated that the rate of change of the permitted flow rate of the liquid through the inlet 14 may have both linearly proportional and non-linearly proportional portions). In other words, when considering the increase of the available cross-sectional area of the inlet 14 as the blocking member 18 is moved from initially open to fully open, the increase is non-linear.

For example, when the blocking member 18 is positioned at a distance x from its closed position, the available cross-sectional area is defined by a function (f(x)). When the blocking member 18 is positioned at a distance 2x from its closed position, the available cross-sectional area is defined by the same function (*f(2x)).* In this example, *f(2x)> 2f(x)* (i.e. as the blocking member 18 is moved towards its open position from an initially open position, each unit of movement results in a small change in the available cross-sectional area of the inlet 14 and as the blocking member moves further the change in the available cross-sectional area becomes larger and larger until the fully open position).

In the present example, the inlet 14 is narrowed at one and becomes wider at an opposing end. The inlet 14 is oriented so that the narrower part becomes open first (as the blocking portion 30 moves outwards along the channel 20). As the blocking portion 30 moves further outwards, the wider parts of the inlet 14 become open. Thus, in this example the rate of change of the flow rate through the valve 10 will increase faster than it would for a rectangular or square inlet. In some embodiments, the inlet 14 has curved corners, which results in a different non-linear change in the flow rate.

The advantage of the inlet 14 being shaped in such a way that the flow rate of the liquid through the valve 10 is initially low and then increases more quickly (relative to a rectangular inlet 14, for example) is that a user can more easily control the direction of liquid flow when only a small amount of liquid is permitted to exit the valve 10. Once the user has established that the direction, etc. of the flow is acceptable they can continue to move the blocking member 18 towards its fully open position (which is reached more quickly due to the shape of the inlet 14). Thus, a valve 10 that has a low liquid flow rate initially (where the blocking member 18 is positioned at a distance x) and increases more quickly due to a wider part of the inlet 14 (where the blocking member is positioned at a distance that is greater than x) is an advantageous arrangement.

The functions that dictated the available cross-sectional area of different shaped inlets are illustrated generally in figures 10a, b and c. A rectangular shape is illustrated in 10a, and as can been seen the increase in available cross-sectional area is linearly proportional with the distance x that the blocking member 18 has travelled. A trapezoidal shape is illustrated in figure 10b. As can be seen the flow rate/available cross-sectional area is small to start with and the increase is steep as the distance *x* the blocking member 18 moves is increased. This results in a slow flow rate initially (to provide an aid for the user) and then approaching the largest flow rate fast as the blocking member 18 is moved further. Thus, the increase in flow rate is proportional to the distance x that the blocking member 18 has moved (but is not linearly proportional).

Figure 10c illustrates a circular shape, which results in a slow increase in the available cross-sectional area initially, then a steep increase as the widest part of the circle is available, followed by a slowing of the increase of the available cross-sectional area at the fully open position.

In the illustrated example, the blocking member 18 "opens" the flow path for liquid through linear movement with respect to the body 12. It should be appreciated that the desired alteration of the flow rate could be achieved using a valve that includes a portion that rotates between its closed position and its open position (and vice versa). The inlet may be positioned on the rotating part and the available cross-sectional area may be increased / decreased as the rotating part rotates. In this case the "distance" that the rotating portion travels through is an arc rather than linear.

It should be appreciated that the details of using, and the functionality of, the valve 10 discussed also apply to the valve 10' which is illustrated in figures 13 - 15b. In that configuration, although the body 12' is formed from first and second body parts 12a', 12b', the way the valve 10' functions once those body parts 12a', 12b' have been connected together is the same as a valve including a single body piece (e.g. the blocking member 18' is inserted into the body passage 20' and moves between open and closed positions in the same manner).

Before the valve 10 is opened to allow waste to flow out of the urostomy appliance 1, the user may connect a conduit or tube 100 to the valve 10, so that the waste flowing out of the urostomy appliance 1 flows into another, connected, receptacle. The receptacle could be a night drainage bag, for example, so that the user does not have to get up during the night to empty their urostomy appliance 1 as it is connected to another (bigger) volume.

Thus, a drainage system is provided by the combination of the urostomy appliance 1 (described above), valve 10 and a connector 102 that is connectable to the conduit 100. Features of the valve 10 that permit the connector 102 to connect to the valve 10 are described below. However, it should be appreciated that another urostomy appliance/valve combination could connect to the connector 102 as long as the valve provided the features outlined here in relation to connecting to the connector 102.

The valve 10 includes the outlet 16 that forms a first fitting having a recess and an internal surface 16a. In some embodiments, the first fitting includes a hollow cylinder, which provides at least part of a flow path out of the valve 10 and into/through the connector 102.

In some embodiments, the first fitting has a circumferentially extending flange portion 52 at or near an entrance to its recess which provides an end face. The flange portion 52 extends generally perpendicularly to an axis A that extends longitudinally through the valve 10.

An embodiment of the connector 102 is illustrated in figure 12. The connector 102 is connected or connectable at a first end to a conduit 100 (the conduit 100 may be connected by way of a spigot or push fit fitting 108, for example). The connector 102 is connected or connectable at a second end to the valve 10. The second end provides a second fitting including a projection 106 having an external surface 106a. In other words, the connector 102 has a main body 104 that extends between the first and second ends.

In some embodiments, the second fitting includes a hollow cylinder, which, in use, provides at least part of a flow path through the connector 102 to the connected conduit 100. In some embodiments, the second fitting has a circumferentially extending shoulder 116 that is spaced from its distal end 106b. The shoulder 116 extends generally perpendicularly to an axis B that extends longitudinally through the connector 102 (although it should be appreciated that this need not be the case as the shoulder may be angled other than at 90 degrees, e.g. generally transversely, to axis B and still provide its function).

In some embodiments, the connector 102 includes two holding formations 110 (although it should be appreciated that there may be more or fewer holding formations 110, as desired). The holding formations 110 are positioned on opposing sides of the main body 104 to each other (in the case of more than two holding formations, they may be spaced around the main body and in the case of one holding formation, it may be positioned to one side). Each holding formation 110 includes an elongate portion 110a that is attached to the main body 104 by an extension portion 110b. The elongate portion 110a extends generally parallel to the axis B and the extension portion 110b extends generally perpendicular to the axis B (away from the main body 104). The extension portion 110a connects to the main body 104 further away from the connector's distal end 106b than the shoulder 116. The elongate portion 110a extends beyond the shoulder 116, towards the distal end 106b, and overlies the second fitting (and, when connected to the valve 10, overlies the flange portion 52). The extension portion 110b connects the elongate portion 110a to the main body 104 with a degree of flexibility so as to provide a pivoting action that allows the elongate portion 110a to move out of general parallel alignment with the main body 104 / axis B.

The elongate portion 110a includes a camming surface 110c. In the illustrated case, the camming surface 110c is positioned at one end of the elongate portion 110a, which is closest to the shoulder 116 (it should be appreciated that there are other arrangements that provide the same functionality). The camming surface 110c faces inwardly, towards the main body 104 / second fitting. This allows the camming surface 110c to engage the flange portion 52 during use (described in more detail below).

The holding formation 110 or at least a part of the holding formation 110 is resiliently biased, so that it may be moved and/or deflected and return to its original position. In the present embodiment, the extension portion 110b provides a pivoting action that allows the elongate portion 110a to deflect outwards. However, it should be appreciated that the holding formation 110 in general is made of a plastics material that is resilient and, therefore, the elongate portion 110a is also able to deform in itself as well as in combination with the extension portion 110b. Thus, it should be appreciated that the extension portion 110b does not necessarily need to provide such a pivoting action in order for the holding formation 110 to function (i.e. hold the connector 102 to the valve 10).

In some embodiments, the second fitting includes a seal 112, which is provided on, connected to or forms part of the external surface 106a. The seal 112 is located in a position between the shoulder 116 and the distal end. In this illustrated case, the seal 112 extends continuously and in an annular shape in a plane that is perpendicular to axis B (but this may not be the case). In some embodiments, the seal 112 is formed using an overmoulding process.

In use, the second fitting (of the connector 102) is received in the first fitting (of the valve 10) when the connector 102 is connected to the valve 10. When the connector 10 is connected to the valve 10, the shoulder 116 engages the end face of the female fitting. Advantageously, the holding formations 110 engage the flange portion 52 such that the connector 102 and the valve 10 are held together and disconnection is inhibited or substantially prevented.

As the connector 102 is moved towards a "connected position", the respective camming surfaces 110a of the holding formations 110 are (automatically) deflected outwards over the flange portion 52. It should be appreciated that if camming surfaces 110a are not provided then a latch formed from a projection maybe present and a user can manually deflect the holding formations 110 over the flange portion 52. Advantageously, if the holding formation 110 is resiliently biased, it moves back to its original position once the camming surfaces have moved past the flange portion 52.

Also when the connector 102 is connected to the valve 10, the seal 112 engages the internal surface 16a of the first fitting. Thus, liquid leakage around the first / second fittings is minimised / inhibited / substantially prevented.

To disconnect the connector 102 from the valve 10, the opposite end of the elongate portion 110a to the camming surface 110c is pressed towards the main body 104, so as to deflect the latch / camming surface 110c over the flange portion 52 (and the connector 102 can be disconnected from the outlet 16 of the valve 10).

It should be appreciated that the conduit 100 has a connector 102 attached at one or both ends for connection to the valve 10 and the night drainage bag, respectively.

When used in this specification and claims, the terms "comprises" and "comprising" and variations thereof mean that the specified features, steps or integers are included. The terms are not to be interpreted to exclude the presence of other features, steps or components.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof.

Although certain example embodiments of the invention have been described, the scope of the appended claims is not intended to be limited solely to these embodiments. The claims are to be construed literally and purposively.

## Claims

1. A valve (10') for a urostomy appliance (1) including:
a body (12') for connection to the urostomy appliance (1) formed from first and second separate body parts (12a', 12b'), which are connected or connectable to each other,
an inlet (14') and an outlet (16'), connected by a flow path, the inlet (14') being provided by the first body part (12a'), and
a blocking member (18')
**characterised in that**
the blocking member (18') is received in an opening provided by the second body part (12b'), said blocking member (18') being moveable between a closed position, in which the blocking member (18') blocks the inlet (14'), such that liquid cannot flow through the flow path to the outlet (16'), and an open position in which the inlet (14') is open, such that liquid is permitted to flow through the flow path to the outlet (16') and
a seal device (70') extends around the blocking member (18') and between the blocking member (18') and the body (12'), which seal device (70') prevents or at least inhibits liquid exiting the valve (10') through the opening in the body (12') and between the body (12') and the blocking member (18').

2. A valve (10') for a urostomy appliance (1) according to claim 1 wherein the seal device (70') extends around an internal surface of the body and optionally, wherein the seal device (70') is connected to the internal surface of the body (12').

3. A valve (10') for a urostomy appliance (1) according to claim 1 or 2 wherein the seal device (70') is located on the second body part (12b') and optionally wherein the seal device (70') is positioned adjacent an entrance of the second body part (12b').

4. A valve (10') for a urostomy appliance (1) according claim 1 or 2 wherein the seal device (70') is located on the first body part (12a') and optionally wherein the seal device (70') is positioned adjacent an entrance of the first body part (12a').

5. A valve (10') for a urostomy appliance (1) according to any of claims 1 to 4 wherein the seal device (70') includes a projection (72') that extends radially inwards.

6. A valve (10') for a urostomy appliance (1) according to any of claims 1 to 5 wherein the seal device (70') includes a portion that extends axially.

7. A valve (10') for a urostomy appliance (1) according to any of claims 6 wherein the portion extends along the flow path in a direction towards the inlet (14').

8. A valve (10') for a urostomy appliance (1) according to any of claims 1 to 7 wherein the seal device (70') includes a projection (72') that extends radially inwards and also extends axially along the passage towards the inlet (20').

9. A valve (10') for a urostomy appliance (1) according to any of the claims 1 to 8 wherein the seal device (70') is formed from a resilient material.

10. A valve (10') for a urostomy appliance (1) according to any of the preceding claims wherein the first body part (12a') includes a first connection formation (60') and the second body part (12b') includes a corresponding second connection formation (62'), such that when the first and second body parts (12a', 12b') are connected the first and second connection formations (60', 62') engage.

11. A valve (10') for a urostomy appliance (1) according to claim 10 wherein one of the first and second connection formations (60') includes a projection (60a') and the other of the first and second connection formations (62') includes a recess (62a').

12. A valve (10') for a urostomy appliance (1) according to claims 10 or 11 wherein the first and second connection formations (60', 62') are connected with an adhesive.

13. A valve (10') for a urostomy appliance (1) according to claims 10 to 12 and at least one of claims 1 to 9 wherein the seal device (70') is positioned adjacent the first or second connection formation (60', 62').

14. A combination of a urostomy appliance (1) and a valve (10'), the urostomy appliance (1) including:
first and second walls (2, 2b) connected at or near their respective peripheries to define an internal waste collecting cavity (4),
a stoma receiving opening (8) which is positioned in the first wall (2a) and is in fluid communication with the waste collecting cavity (4), and
the valve (10') according to any of claims 1 to 13 connected to one of the first and second walls (2a, 2b).

## Patentansprüche

1. Ventil (10') für ein Urostomiegerät (1), beinhaltend:
einen Körper (12') zum Anschluss an das Urostomiegerät (1), gebildet aus
einem ersten und einem zweiten separaten Körperteil (12a', 12b'), die miteinander verbunden oder verbindbar sind,
einen Einlass (14') und einen Auslass (16'), die durch einen Strömungsweg verbunden sind, wobei der Einlass (14') von dem ersten Körperteil (12a') bereitgestellt wird, und
ein Blockierelement (18'),
**dadurch gekennzeichnet, dass**
das Blockierelement (18') in einer Öffnung aufgenommen wird, die von dem zweiten Körperteil (12b') bereitgestellt wird, wobei das Blockierelement (18') bewegbar ist zwischen einer geschlossenen Position, in der das Blockierelement (18') den Einlass (14') blockiert, sodass Flüssigkeit nicht durch den Strömungsweg zu dem Auslass (16') fließen kann, und einer offenen Position, in der der Einlass (14') offen ist, sodass Flüssigkeit durch den Strömungsweg zu dem Auslass (16') fließen kann, und
eine Dichtungsvorrichtung (70') sich um das Blockierelement (18') und zwischen dem Blockierelement (18') und dem Körper (12') erstreckt, wobei diese Dichtungsvorrichtung (70') verhindert oder zumindest hemmt, dass Flüssigkeit aus dem Ventil (10') durch die Öffnung in dem Körper (12') und zwischen dem Körper (12') und dem Blockierelement (18') austritt.

2. Ventil (10') für ein Urostomiegerät (1) nach Anspruch 1, wobei sich die Dichtungsvorrichtung (70') um eine Innenfläche des Körpers erstreckt und optional, wobei die Dichtungsvorrichtung (70') mit der Innenfläche des Körpers (12') verbunden ist.

3. Ventil (10') für ein Urostomiegerät (1) nach Anspruch 1 oder 2, wobei sich die Dichtungsvorrichtung (70') auf dem zweiten Körperteil (12b') befindet und optional, wobei die Dichtungsvorrichtung (70') benachbart zu einem Eingang des zweiten Körperteils (12b') positioniert ist.

4. Ventil (10') für ein Urostomiegerät (1) nach Anspruch 1 oder 2, wobei sich die Dichtungsvorrichtung (70') auf dem ersten Körperteil (12a') befindet und optional, wobei die Dichtungsvorrichtung (70') benachbart zu einem Eingang des ersten Körperteils (12a') positioniert ist.

5. Ventil (10') für ein Urostomiegerät (1) nach einem der Ansprüche 1 bis 4, wobei die Dichtungsvorrichtung (70') einen Vorsprung (72') beinhaltet, der sich radial nach innen erstreckt.

6. Ventil (10') für ein Urostomiegerät (1) nach einem der Ansprüche 1 bis 5, wobei die Dichtungsvorrichtung (70') einen Abschnitt beinhaltet, der sich axial erstreckt.

7. Ventil (10') für ein Urostomiegerät (1) nach Anspruch 6, wobei sich der Abschnitt entlang des Strömungswegs in einer Richtung hin zu dem Einlass (14') erstreckt.

8. Ventil (10') für ein Urostomiegerät (1) nach einem der Ansprüche 1 bis 7, wobei die Dichtungsvorrichtung (70') einen Vorsprung (72') beinhaltet, der sich radial nach innen erstreckt und sich auch axial entlang des Durchgangs hin zu dem Einlass (20') erstreckt.

9. Ventil (10') für ein Urostomiegerät (1) nach einem der Ansprüche 1 bis 8, wobei die Dichtungsvorrichtung (70') aus einem resilienten Material gebildet ist.

10. Ventil (10') für ein Urostomiegerät (1) nach einem der vorhergehenden Ansprüche, wobei der erste Körperteil (12a') eine erste Verbindungsformation (60') beinhaltet und der zweite Körperteil (12b') eine entsprechende zweite Verbindungsformation (62') beinhaltet, sodass, wenn der erste und zweite Körperteil (12a', 12b') verbunden sind, die erste und die zweite Verbindungsformation (60', 62') in Eingriff treten.

11. Ventil (10') für ein Urostomiegerät (1) nach Anspruch 10, wobei eine der ersten und der zweiten Verbindungsformation (60') einen Vorsprung (60a') beinhaltet und die andere der ersten und der zweiten Verbindungsformation (62') eine Aussparung (62a') beinhaltet.

12. Ventil (10') für ein Urostomiegerät (1) nach Anspruch 10 oder 11, wobei die erste und die zweite Verbindungsformation (60', 62') mit einem Klebstoff verbunden sind.

13. Ventil (10') für ein Urostomiegerät (1) nach Anspruch 10 bis 12 und mindestens einem der Ansprüche 1 bis 9, wobei die Dichtungsvorrichtung (70') benachbart zu der ersten oder zweiten Verbindungsformation (60', 62') positioniert ist.

14. Kombination aus einem Urostomiegerät (1) und einem Ventil (10'), wobei das Urostomiegerät (1) beinhaltet:
eine erste und eine zweite Wand (2, 2b), die an oder nahe ihren jeweiligen Peripherien verbunden sind, um einen internen Ausscheidungssammelhohlraum (4) zu definieren,
eine Stomaaufnahmeöffnung (8), die in der ersten Wand (2a) positioniert ist und in Fluidkommunikation mit dem Ausscheidungssammelhohlraum (4) steht, und
das Ventil (10') nach einem der Ansprüche 1 bis 13, das mit einer der ersten und der zweiten Wand (2a, 2b) verbunden ist.

## Revendications

1. Vanne (10') pour appareil d'urostomie (1) comprenant :
un corps (12') destiné à être relié à l'appareil d'urostomie (1) formé à partir de :
première et seconde parties de corps séparées (12a', 12b'), qui sont reliées ou peuvent être reliées l'une à l'autre,
une entrée (14') et une sortie (16'), reliées par un trajet d'écoulement, l'entrée (14') étant assurée par la première partie de corps (12a'), et
un élément de blocage (18'),
la vanne étant **caractérisée en ce que** :
l'élément de blocage (18') est reçu dans une ouverture formée par la seconde partie de corps (12b'), ledit élément de blocage (18') étant mobile entre une position fermée dans laquelle l'élément de blocage (18') bloque l'entrée (14'), de telle sorte qu'un liquide ne puisse pas s'écouler par le trajet d'écoulement jusqu'à la sortie (16'), et une position ouverte dans laquelle l'entrée (14') est ouverte, de telle sorte qu'un liquide puisse s'écouler par le trajet d'écoulement jusqu'à la sortie (16'), et
un dispositif d'étanchéité (70') s'étend autour de l'élément de blocage (18') et entre l'élément de blocage (18') et le corps (12'), ledit dispositif d'étanchéité (70') évitant ou au moins empêchant le liquide de sortir de la vanne (10') par l'ouverture dans le corps (12') et entre le corps (12') et l'élément de blocage (18').

2. Vanne (10') pour appareil d'urostomie (1) selon la revendication 1, le dispositif d'étanchéité (70') s'étendant autour d'une surface interne du corps et, éventuellement, le dispositif d'étanchéité (70') étant relié à la surface interne du corps (12').

3. Vanne (10') pour appareil d'urostomie (1) selon la revendication 1 ou 2, le dispositif d'étanchéité (70') étant situé sur la seconde partie de corps (12b') et, éventuellement, le dispositif d'étanchéité (70') étant positionné adjacent à une entrée de la seconde partie de corps (12b').

4. Vanne (10') pour appareil d'urostomie (1) selon la revendication 1 ou 2, le dispositif d'étanchéité (70') étant situé sur la première partie de corps (12a') et, éventuellement, le dispositif d'étanchéité (70') étant positionné adjacent à une entrée de la première partie de corps (12a').

5. Vanne (10') pour appareil d'urostomie (1) selon l'une quelconque des revendications 1 à 4, le dispositif d'étanchéité (70') comprenant une saillie (72') qui s'étend radialement vers l'intérieur.

6. Vanne (10') pour appareil d'urostomie (1) selon l'une quelconque des revendications 1 à 5, le dispositif d'étanchéité (70') comprenant une partie qui s'étend axialement.

7. Vanne (10') pour appareil d'urostomie (1) selon la revendication 6, la partie s'étendant le long du trajet d'écoulement dans une direction vers l'entrée (14').

8. Vanne (10') pour appareil d'urostomie (1) selon l'une quelconque des revendications 1 à 7, le dispositif d'étanchéité (70') comprenant une saillie (72') qui s'étend radialement vers l'intérieur et qui s'étend également axialement le long du passage vers l'entrée (20').

9. Vanne (10') pour appareil d'urostomie (1) selon l'une quelconque des revendications 1 à 8, le dispositif d'étanchéité (70') étant formé à partir d'un matériau élastique.

10. Vanne (10') pour appareil d'urostomie (1) selon l'une quelconque des revendications précédentes, la première partie de corps (12a') comprenant une première formation de liaison (60') et la seconde partie de corps (12b') comprenant une seconde formation de liaison (62') correspondante, de telle sorte que lorsque les première et seconde parties de corps (12a', 12b') sont reliées, les première et seconde formations de liaison (60', 62') entrent en prise.

11. Vanne (10') pour appareil d'urostomie (1) selon la revendication 10, l'une des première et seconde formations de liaison (60') comprenant une saillie (60a') et l'autre des première et seconde formations de liaison (62') comprenant un évidement (62a').

12. Vanne (10') pour appareil d'urostomie (1) selon la revendication 10 ou 11, les première et seconde formations de liaison (60', 62') étant reliées par un adhésif.

13. Vanne (10') pour appareil d'urostomie (1) selon les revendications 10 à 12 et au moins l'une des revendications 1 à 9, le dispositif d'étanchéité (70') étant positionné adjacent à la première ou à la seconde formation de liaison (60', 62').

14. Combinaison d'un dispositif d'urostomie (1) et d'une vanne (10'), le dispositif d'urostomie (1) comprenant :
des première et seconde parois (2, 2b) reliées au niveau ou à proximité de leurs périphéries respectives pour définir une cavité interne de collecte de déchets (4),
une ouverture de réception de stomie (8) qui est positionnée dans la première paroi (2a) et qui est en communication fluidique avec la cavité de collecte de déchets (4), et
la vanne (10') selon l'une quelconque des revendications 1 à 13, reliée à l'une des première et seconde parois (2a, 2b).
